Europäisches Patentamt

European Patent Office

Office européen des brevets

(11) Veröffentlichungsnummer: **0 296 544**
**A2**

## (12) EUROPÄISCHE PATENTANMELDUNG

(21) Anmeldenummer: 88109886.7

(22) Anmeldetag: 22.06.88

(51) Int. Cl.⁴: **G01N 33/543 , G01N 33/577 , G01N 33/574 , G01N 33/78**

(30) Priorität: 25.06.87 DE 3720983

(43) Veröffentlichungstag der Anmeldung:
28.12.88 Patentblatt 88/52

(84) Benannte Vertragsstaaten:
AT BE CH DE ES FR GB GR IT LI LU NL SE

(71) Anmelder: **HOECHST AKTIENGESELLSCHAFT**
**Postfach 80 03 20**
**D-6230 Frankfurt am Main 80(DE)**

(72) Erfinder: **Schnorr, Gerd, Dr.**
**Auf dem Lattigkopf 23**
**D-6368 Bad Vilbel(DE)**
Erfinder: **Strecker, Helmut, Dr.**

**Verstorben(DE)**
Erfinder: **Molz, Peter, Dr.**
**Kaiser-Wilhelm-Ring 44**
**D-6500 Mainz(DE)**
Erfinder: **Simons, Guido, Dr.**
**Talstrasse 24**
**D-6507 Ingelheim am Rhein(DE)**
Erfinder: **Skrzipczyk, Heinz Jürgen, Dr.**
**Am Schellberg 16**
**D-6232 Bad Soden am Taunus(DE)**
Erfinder: **Harthus, Hans-Peter, Dr.**
**Auf der Jöch 8**
**D-3550 Marburg(DE)**
Erfinder: **Walter, Götz, Dr.**
**Zur Hainbuch 12**
**D-3550 Marburg-Moischt(DE)**

(54) **Immunometrisches Bestimmungsverfahren.**

(57) Gegenstand der Erfindung ist ein immunometrisches Bestimmungsverfahren, bei dem zur Vermeidung unspezifisch erhöhter oder erniedrigter Werte für das zu bestimmende Antigen dem unmarkierten (1) und/oder dem markierten Antikörper (2) im Überschuß eine Substanz (Suppressions-Substanz) (6) zugesetzt wird, welche aufgrund ihrer großen Ähnlichkeit mit den verwendeten Reagenz-Antikörpern und aufgrund ihres hohen Überschusses, die mit den Antikörpern interferierende Substanz (3) selektiv abfängt und somit eine Beeinflussung des Assays ausschließt.

FIG.3

## Immunometrisches Bestimmungsverfahren

Gegenstand der Erfindung ist ein immunometrisches Bestimmungsverfahren, bei dem zur Vermeidung unspezifisch erhöhter oder erniedrigter Werte für das zu bestimmende Antigen dem unmarkierten und/oder dem markierten Antikörper im Überschuß eine Substanz (Suppresions-Substanz) zugesetzt wird, welche aufgrund ihrer großen Ähnlichkeit mit den verwendeten Reagenz-Antikörpern und aufgrund ihres hohen Überschusses, die mit den Antikörpern interferierende Substanz selektiv abfängt und somit eine Beeinflussung des Assays ausschließt.

Es ist bekannt, daß zum qualitativen und quantitativen Nachweis von Antigenen in immer größerem Maß immunometrische Methoden herangezogen werden. Diese Methoden beruhen auf der Bildung eines Komplexes des Antigens mit einem oder mehreren Antikörpern, wobei einer der Bindungspartner so markiert ist, daß er mittels physikalischer oder chemischer Nachweisverfahren qualitativ und/oder quantitativ erfaßt werden kann. Dadurch ist es möglich festzustellen, ob und in welcher Menge sich ein Komplex aus dem Antigen und einem oder mehreren Antikörpern gebildet hat. Entschiedende Verbesserungen der immunometrischen Bestimmungsverfahren gelangen mit der Einführung monoklonaler Antikörper (Milstein und Köhler, 1975), deren Anwendung in immunometrischen Assays in der deutschen Offenlegungsschrift 31 30 834 eingehend beschrieben ist.

Die immunometrischen Bestimmungsmethoden können in zwei Haupttypen unterteilt werden, je nachdem ob das Antigen oder der Antikörper markiert ist. Der markierte Bindungspartner wird stets im Überschuß verwendet.

Besonderes Interesse haben immunometrische Verfahren gefunden, bei denen einer der verwendeten Antikörper markiert ist. Dabei wird das Antigen in Form eines ternären Komplexes sandwichartig gebunden und nach der Inkubation der nichtgebundene markierte Antikörper durch Dekantieren oder Auswaschen entfernt. Diese Ausführungsformen werden je nach der Art der Markierung als zweiseitiger immunoradiometrischer Assay (IRMA), als immuno-enzymometrischer Assay (IEMA) oder immuno-chemiluminometrischer Assay (ICMA) bezeichnet. In der Regel ist dabei der unmarkierte Antikörper an eine feste Phase gebunden.

Die vorstehend genannten Sandwich-Assays können in verschiedenen Varianten zur Ausführung kommen, die sich durch die zur Bildung des ternären Komplexes führenden Reaktionsschritte unterscheiden. Man kann entweder in einem Einschrittverfahren das Antigen mit dem markierten und dem unmarkierten Antikörper gleichzeitig zusammengeben, man kann aber auch sequentiell vorgehen und das Antigen zunächst mit dem unmarkierten und nach einer ausreichenden Inkubationszeit dann auch mit dem markierten Antikörper reagieren lassen. Schließlich können diese Reaktionsschritte auch in der umgekehrten Reihenfolge durchgeführt werden.

Die auf der Verwendung von markierten Antikörpern beruhenden Sandwich-Assays haben gegenüber den unter Verwendung markierter Antigene durchgeführten Assays entscheidende analytische Vorteile: Es ist möglich, die Antikörper im Überschuß einzusetzen und durch die Erhöhung ihrer Konzentration das Gleichgewicht in Richtung auf die Bildung des ternären Komplexes zu verschieben. Damit können auch noch geringe Antigenmengen gebunden und an die Markierung gekoppelt werden. Da außerdem das von der Markierung ausgesendete Signal der Antigenkonzentration direkt proportional ist und die Signal-Dosis-Kurve steil verläuft, können auch die durch niedrige Antigenkonzentrationen verursachten schwachen Signale noch deutlich erkannt werden. Aus diesen Gründen sind die mit markierten Antikörpern arbeitenden Sandwich-Assays wesentlich empfindlicher als Assays, die auf der Verwendung markierter Antigene beruhen.

Schließlich spricht für die mit markierten Antikörpern arbeitenden Sandwich-Assays auch der wesentlich größere dynamische Meßbereich, d.h. es gibt einen größeren Bereich, in welchem sich bei einer Änderung der Konzentration des Antigens auch die Stärke des ausgesendeten Signals des ternären Komplexes empfindlich ändert. Außerdem ist es für die routinemäßige Anwendung kommerzieller immunometrischer Bestimmungsverfahren noch von Bedeutung, daß sie möglichst kurze Inkubationszeiten gestatten. Das ist dann gewährleistet, wenn höhere Reagenzkonzentrationen eingesetzt werden können, wie es bei dem Einsatz von markierten Antikörpern in Sandwich-Assays der Fall ist.

Beide Assay-Typen (solche mit markierten Antikörpern und solche mit markierten Antigenen) können durch Serumbestandteile, welche in die primäre Antigen-Antikörper-Reaktion und/oder Trennreaktion eingreifen, gestört werden. Unter Trennreaktion wird hierbei die Auftrennung von gebundenem und freiem Tracer verstanden. Dabei können je nach Art der Störung und Prinzip des Assay entweder falsch erhöhte oder falsch erniedrigte Werte erhalten werden. Trennungen, die auf unspezifischen Fällungsreaktionen beruhen, wie beispielsweise die IgG-Ausfällung durch Alkohole wie Polyethylenglycol (PEG), besitzen natürlich den größten Robustheitsgrad in Bezug auf immunologische Störeinflüsse, führen andererseits aber in Folge ihrer stark schwankenden NSB (nicht-spezifische Bindung)-Anteile (Mißklassifikation von freiem Tracer) häufig zu analytisch/diagnostisch unsicheren Aussagen. Die sich durch niedrige und präzise NSB auszeichnenden Doppelantikörpertrenntechniken werden durch körpereigene heterophile Antikörper, welche gegen die primären (antigenbindenden) Antikörper gerichtet sind, in der Weise gestört, daß der Trennantikörper nicht mehr an dem Antigen-Antikörper-Komplex angreifen kann. Als Folge hiervon wird in kompetitiven Assays der Antigengehalt in der Patientenprobe überschätzt (A. Sain et al. 71, 540 (1979)).

Interferenzen bei der primären Antigen-Antikörper-Reaktion wurden häufig bei dem Auftreten von Auto-Antikörpern gegen den Analyten beobachtet. Bei Doppelantikörper-RIAs führt dieser Effekt zu einem falsch erhöhten Ergebnis, während bei den PEG-Assays falsch erniedrigte Werte erhalten werden. Eine durch Vorfällung mit PEG durchgeführte Probenvorbereitung vermeidet diese Art der Interferenz.

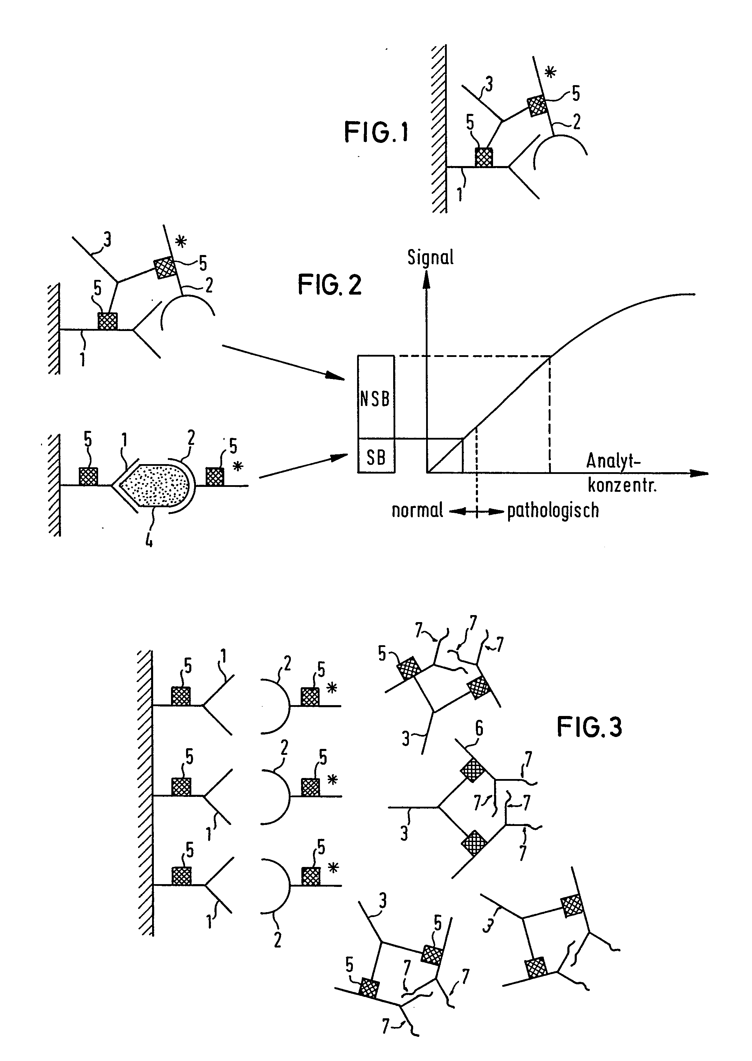

Bei den Sandwich-Assays wurden in jüngster Zeit ebenfalls Interferenzen beobachtet, die sich dadurch manifestieren, daß durch eine Erhöhung der NSB Patientenseren falsch zu hoch gemessen werden. Als Ursache werden Antikörper gegen die Reagenzien der Assays vermutet (R.J. Thompson et al., Clin. Chem. 32, 476 (1986) (Fig. 1 und 2).

In Figur 1 ist dies anhand der in der Literatur üblichen Darstelungsweise für Antikörper dargestellt. Ein festphasengebundener Antikörper (1) wird über eine nicht-analytspezifische Bindung (NSB), beispielsweise durch eine Interferenzsubstanz (3) an dem Epitop (5) eines markierten Antikörpers (2) gebunden.

In Figur 2 ist in einem Diagramm dargestellt, wie sich solche nicht-spezifischen Bindungen (NSB) zu den spezifischen Bindungen (SB) zwischen festphasengebundenem Antikörper (1), Antigen bzw. Analyt (4) und markiertem Antikörper (2) addieren und so zu hohe, oft pathologische Analytkonzentrationen vortäuschen.

Da es sich bei den Interferenzsubstanzen um spezies-spezifische Antikörper handelt, können diese nicht durch jedwedes IgG gebunden werden (wie etwa bei Rheumafaktoren), sondern es muß speziesspezifisches Träger-Gammaglobulin eingesetzt werden.

Nach dem gegenwärtigen Stand der Technik wird Assays, bei denen monoclonale Antikörper von der Maus verwendet werden, unspezifisches Maus- oder Ratten-Serum zugesetzt (EP-A 0 174 026 oder RIA-gnost (R)hCG, Behring-Werke, Marburg, seit 1.9.1984). Jedoch treten trotz dieser Maßnahme immer wieder Seren auf, bei denen aufgrund des hohen Titers an Anti-Maus-IgG falsch erhöhte Werte gefunden werden. Werden diese Proben durch Zusatz einer größeren Menge Maus-oder Ratten-Serum individuell nachgearbeitet, tritt im allgemeinen eine Erniedrigung des Initial-Wertes auf. Die hierfür erforderlichen großen Mengen verbieten einen Einsatz in dem Assay als prophylaktische Maßnahme, da eine Beeinflussung des Assays zu erwarten ist.

Erfindungsgemäß können die oben beschriebenen Nachteile dadurch beseitigt werden, daß man als Träger-(Suppressions)-Substanz (6) ein Protein verwendet, welches den im Assay verwendeten IgGs immunologisch sehr ähnlich ist und im Idealfall nur in der Antigenbindungsstruktur (7) von diesen abweicht und somit den Analyten nicht bindet (Fig. 3). Diese Substanzen können in großem Überschuß dem markierten Reagenzantikörper beigemischt werden, ohne in die Analyt-Reaktion einzugreifen.

Voraussetzung hierfür ist des weiteren eine immunologische Indifferenz gegenüber allen übrigen Serumbestandteilen. Die Nicht-Erfüllung dieser Forderung könnte zu Netzwerkbildung in der Serumprobe führen und die suppressive Wirkung der Substanz verringern. Außerdem könnte ein Einfluß auf die Kinetik und Gleichgewichtslage der Analytreaktion nicht ausgeschlossen werden.

Gegenstand der Erfindung ist deshalb ein immunometrisches Bestimmungsverfahren für eine mindestens zwei Antikörperbindungsstellen aufweisende antigene Substanz, bei dem man eine die antigene Substanz (a) enthaltende Flüssigkeitsprobe mit einem auf einer Festphase immobilisierten, unmarkierten, für (a) spezifischen Antikörper (b) und einem weiteren, für (a) spezifischen markierten Antikörper (c) sequentiell oder in einem Schritt inkubiert, wobei sich ein festphasengebundener, ternärer Komplex aus (a), (b) und (c) bildet, der ein nachweisbares Signal entsprechend der Menge von (a) aussendet, das dadurch gekennzeichnet ist, daß bei dem sequentiellen Verfahren im ersten und/oder zweiten Inkubationsschritt, bei dem 1-Schritt-Verfahren zu dem Reaktionsgemisch, Protein zugesetzt wird, welches den verwendeten Antikörpern immunologisch verwandt ist und welches damit in der Lage ist, im Patientenserum vorhandene Komponenten, die mit den Testreagenzien unerwünschte Bindungen eingehen würden, zu binden, un damit eine negative Testbeeinflussung auszuschließen.

Bevorzugt handelt es sich bei den eingesetzten Proteinen um einen oder mehrere verschiedene Antikörper, insbesondere um solch einen/solche der gleichen Spezies (Tierspezies) wie die der verwendeten Reagenzantikörper. Besonders bevorzugt sind hierbei monoclonale Antikörper, ganz besonders bevorzugt anti-idiotypische monoclonale Antikörper. Unter Protein wird im Vorstehenden und Nachfolgenden eine makromolekulare organische Verbindung, aufgebaut aus Aminosäureeinheiten verstanden, die antigene Eigenschaften besitzt.

Unter anti-idiotypischen Antikörpern werden solche verstanden, die gegen die idiotype Region des Antikörpers gerichtet sind, der ihre Bildung hervorgerufen hat.

Bevorzugt geht man bei der Herstellung der erfindungsgemäßen Suppressions-Substanzen von den verwendeten Reagenz-Antikörpern aus und verändert diese so, daß sie zwar noch ihre Spezifität gegen die Interferenz-Substanz bewahren, jedoch nicht mehr - oder zumindest deutlich vermindert -mit dem zu analysierenden Antigen reagieren. Durch den hohen Überschuß, in dem diese Suppressions-Substanzen zweckmäßiger Weise dem immunometrischen Assay zugesetzt werden, wird von den beiden Konkurenzreaktionen -Reagenzantikörper + interferierende Substanz und Suppressions-Substanz + interferierende Substanz - die letztere bei weitem bevorzugt, so daß die interferierenden Substanzen nahezu quantitativ aus dem Reaktionsgleichgewicht entfernt werden.

Zur Erzeugung dieser Suppressions-Substanzen bieten sich -ausgehend von den Reagenz-Antikörpern - beispielsweise folgende Möglichkeiten an:

a) Selektive Abspaltung der Antigen-bindenden Strukturen des verwendeten Reagenz-Antikörpers

b) Blockade der Antigen-bindenden Strukturen des verwendeten Reagenz-Antikörpers durch eine komplementäre Antigen-Struktur

c) Abwandlung der Antigen-bindenden Struktur des verwendeten Reagenz-Antikörpers durch äußeren Eingriff beispielsweise durch Mutation in der Zellkultur

d) Herstellung anti-idiotypischer Antikörper (s. US-Patentschrift 4 536 479), die der gleichen IgG-Subklasse angehören wie die Reagenz-Antikörper

Gemäß Variante a) lassen sich nach literaturbekannten Verfahren Antikörper beispielsweise mittels Enzymen spalten. Hierbei ist es möglich einzelne Fragmente sowohl von den leichten als auch von den - schweren Ketten abzuspalten. Bekanntlich befinden sich die antigenbindenden Strukturen am N-terminalen Ende der Polypeptidketten, so daß mit deren Abspaltung die epitoperkennenden Bestandteile des Antigens fast vollständig verloren gehen.

Ebenso ist es möglich die beiden Fab-Teile von dem Fc-Teil zu trennen. Liegen beispielsweise die Epitope, die von der interferierenden Substanz erkannt werden, auf dem Fc-Teil des Testantikörpers, so wäre der reine Fc-Teil dieses Antikörpers ein ideales Suppressionsmedium, da es zwar mit der Interferenzsubstanz reagiert, aufgrund der nicht vorhandenen Fab-Teile jedoch nicht mit den Antigen (Analyt).

Die Blockade der antigenbindenden Strukturen des verwendeten Reagenzantikörpers durch eine komplementäre Antigen-Struktur gemäß b) kann generell durch jede Substanz erfolgen, die gegen die idiotype Region des Reagenzantikörpers gerichtet ist und deren Bindungsaffinität genügend groß ist, beispielsweise durch einen anti-idiotypischen Antikörper.

Die Abwandlung durch Mutation in der Zellkultur gemäß c) kann beispielsweise folgendermaßen erfolgen:

Aus Zellkulturen werden somatische Mutanten von monoklonalen Antikörpern isoliert und anschließend die Effekte dieser Mutationen auf die Antikörperfunktion überprüft. Die Isolierung solcher Mutanten mit veränderter Ig-Struktur wird durch die Instabilität der Ig-Gene in kultivierten Hybridom-Zellen ermöglicht (Morrison et al., CRC Crit. Rev. Immunol, 3: 1 - 22, 1981). Man kennt 3 Arten von strukturellen Mutanten mit besonderer Bedeutung für die Hybridom-Technologie: (1) Klassen- und Subklassen-Switch-Varianten (Cebra et al., Ann. Rev. Immunol., 2: 493 - 548, 1984; Sublitzky et al., Immunol. Rev., 67: 59 - 72, 1982; Shimizu et al., Cell, 36: 801 - 803, 1984; Tilley et al. Proc. Natl. Acad. Sci., USA, 80: 6967 - 6971, 1983); (2) Deletions-oder Punktmutationen in der konstanten Region mit Änderungen der Effektorfunktion (Yelton et al., J. Exp. Med., 156: 1131 - 1148, 1982; Kenter et al., Science, 206: 1307 - 1309, 1979; Teillaud et al., J. Immunol, 1984 in press); und (3) Mutanten mit veränderten Antigenbindungseigenschaften (Dildrop et al., EMBO J., 1: 635 - 640, 1982; Cook et al., Proc. Natl. Acad. Sci. USA, 74: 5687 - 5691, 1977). Solche Mutationen, welche letztlich zum kompletten Verlust der Fähigkeit führen, ein spez. Antigen zu binden, treten sehr häufig spontan bei einigen Klonen auf und rühren her von der Substitution einzelner Aminosäuren in der schweren Kette der V-Region (Rudikoff et al., Proc. Natl. Acad. Sci USA, 79: 197 - 1983, 1982).

Für den Einsatz als Suppressionsmedium kommt bevorzugt Variante 3 in Frage. Die Auswahl der am besten geeigneten Mutanten erfordert lediglich einige abklärende Versuche, bei denen jeweils festgestellt wird ob

1. Die antigenbindenden Eigenschaften noch vorhanden sind und

2. ob die interferierende Substanz "ihre" Epitope auf der Mutante noch erkennt und somit bindet.

Gemäß d) können anti-idiotypischen Antikörper durch Immunisierung mit einem monoklonalen Antikörper - gerichtet gegen ein Nicht-Analyt-Antigen - erzeugt werden. Diese Substanzen weisen Epitop-Homologie in Bezug auf die konstanten Regionen des Reagenzantikörpers auf und erfüllen die Forderung nach Nicht-Reaktion mit den übrigen Serumbestandteilen.

Die gemäß a-d hergestellten Suppressions-Sustanzen können in immunometrischen Bestimmungsverfahren eingesetzt werden, mit dem Ziel, interferierende Stoffe (z.B. Antikörper o.ä.) selektiv abzufangen. Sie sind einsetzbar in Immuno-Assays zur Bestimmung der unterschiedlichsten antigenen Substanzen, z.B. für das humane thyreoideastimulierende Hormon (hTSH) oder für die onkofetalen Proteine wie Alfa-Fetoprotein (AFP), humanes Choriongonadotropin (hCG) oder das carcinoembryonale Antigen (CEA). Besonders vorteilhaft ist die Verwendung bei Analyten (Antigenen), die in niedrigen Konzentrationen vorkommen oder bei Gesunden praktisch nicht auftreten und bei denen bereits geringe Konzentrationen einen pathologischen Zustand anzeigen. In diesen Fällen würden dann auch schon geringe, durch Interferenz hervorgerufene Konzentrationserhöhungen bereits einen pathologischen Zustand anzeigen.

Das neue erfindungsgemäße immunometrische Bestimmungsverfahren ist zuverlässig, technisch einfach, schnell und robust. Es kann bei einer Vielzahl von kommerziellen Sandwich-Bestecken angewendet werden.

Suppressions-Substanzen der zuvor beschriebenen Eigenschaften können auch in bestehende kommerzielle Testbestecke nachträglich eingebracht werden.

Sie können dazu zweckmäßigerweise in mindestens dem 10-, bevorzugt dem 50-, besonders bevorzugt dem 80-fachen und höchstens dem 1000-, bevorzugt dem 500-, besonders bevorzugt dem 120-fachen der Reagenzantikörperkonzentrationen, den herkömmlichen Assays zugesetzt werden.

Bei den kommerziellen Testbestecken handelt es sich bevorzugt um solche, bei denen der festphasengebundene poly-, bevorzugt jedoch monoklonale Antikörper, unmarkiert ist. Dieser Antikörper wird bevorzugt adsorptiv oder kovalent an die Festphase gebunden, wobei die Festphase bevorzugt aus einem Kunststoffröhrchen, einer Mikrotiterplatte, Kunststoffgegenständen wie Kunststoffkugeln oder Kunststoffpropellern oder auch aus mikroskopisch kleinen Kunststoffkugeln, die beispielsweise in einer Flüssigkeit suspendiert sind, besteht. Bei den markierten Antikörpern handelt es sich ebenfalls um poly-, bevorzugt jedoch um monoklonale Antikörper. Die Markierung erfolgt nach literaturbekannten Verfahren, mittels eines radioaktiven Isotops, eines Enzyms oder einer fluoreszierenden oder chemilumineszierenden Gruppe.

Die Erfindung wird durch die nachfolgenden Beispiele näher erläutert.

Beispiel 1

Herstellung somatischer Mutanten von monoklonalen Antikörpern

Hybridomzellklone, welche Antikörper bekannter Spezifität produzieren, wurden mit Hilfe eines Single-Cell-Manipulators oder des Limiting-Dilution-Verfahrens in Mikrotiterplatten rekloniert. Die Kulturüberstände aus solchen Wells mit positivem Zellwachstum wurden dann zunächst auf ihren Ig-Gehalt und anschließend auf ihre Antigenbindungseigenschaften getestet. Bei genügend hoher Klonierungsrate erhielt man Zellklone, welche sich durch eine hohe Produktion von Ig-Molekülen bei gleichzeitiger Antigenbindungsinsuffizienz auszeichneten.

Beispiel 2

Herstellung monoklonaler Antiidiotypischer Antikörper

Zur Herstellung monoklonaler anti-Idiotyp Antikörper wurde ein monoklonaler Antikörper als Immunisierungsantigen verwendet. In diesem Falle wurde ein gegen das carcinoembryonale Antigen (CEA) gerichteter monoklonaler Antikörper syngener Herkunft benutzt, welcher als Ganzantikörper weiblichen, 6 - 8 Wochen alten BALB/c-Mäusen appliziert wurde. Hierbei wurden pro Maus ca. 10 µg des Ganzantikörpers, emulgiert in komplettem Freund'schen Adjuvants, subcutan und in einem zweiten Falle intraperitoneal injiziert. Jeweils 4 bzw. 8 Wochen später folgte eine zweite und dritte Immunisierung. Unmittelbar vor der eigentlichen Fusion wurden die Versuchstiere zusätzlich 4 Tage hintereinander intravenös geboostert. Am Tage der Fusion wurden die Milzen steril entnommen und zu Einzelzellen suspendiert. Durch Fusion von $10^8$ Milzzellen mit $2 \times 10^7$ Zellen einer Myelomzellinie (SP 2/0) wurden Hybridzellen erzeugt, welche anschließend in einem Selektionsmedium (DMEM (Dulbecco's minimal essential Medium) + 20 % FKS (fetales Kälberserum); 0,1 mM Hypoxanthin; 0,4 mM Aminopterin, 16 mM Thymidin) auf 24-Well Platten (Costar), in einer Konzentration von $10^6$ Zellen/Well, ausgesät wurden. 2 - 3 Wochen später wurden aus den Wells einzelne Zellkolonien isoliert und in jeweils ein anderes Well neuer Kulturplatten (24-Well, Costar) übertragen. Nach weiteren 2 - 3 Tagen wurden diese Kulturüberstände in einem Enzymimmunoassay, unter Verwendung des Immunisierungsantikörpers konjugiert mit Peroxidase (POD), auf vorhandene anti-Idiotyp Antikörper und diese wiederum in einem weiteren Enzymimmunoassay auf ihre Antigenhemmbarkeit überprüft. Antigenhemmbare anti-Idiotyp Antikörper produzierende Hybride wurden selektioniert und mit Hilfe eines Single-Cell-Manipulators kloniert.

Beispiel 3

Analog Beispiel 2 wurden anti-idiotypische Antikörper hergestellt, bei der zur Immunisierung an Stelle des Ganzantikörpers das Fab'-Fragment des gegen CEA gerichteten Antikörpers, gekoppelt an BSA, verwendet wurde.

Beispiel 4

Analog Beispiel 2 wurden anti-idiotypische Antikörper hergestellt, bei denen zur Immunisierung an Stelle des Ganzantikörpers das Fab'-Fragment des gegen CEA gerichteten Antikörpers, gekoppelt an KLH, verwendet wurde.

Zum Nachweis der Suppressionseigenschaften der erfindungsgemäßen Substanzen wurden folgende vergleichende Versuche durchgeführt:

Beispiel 5

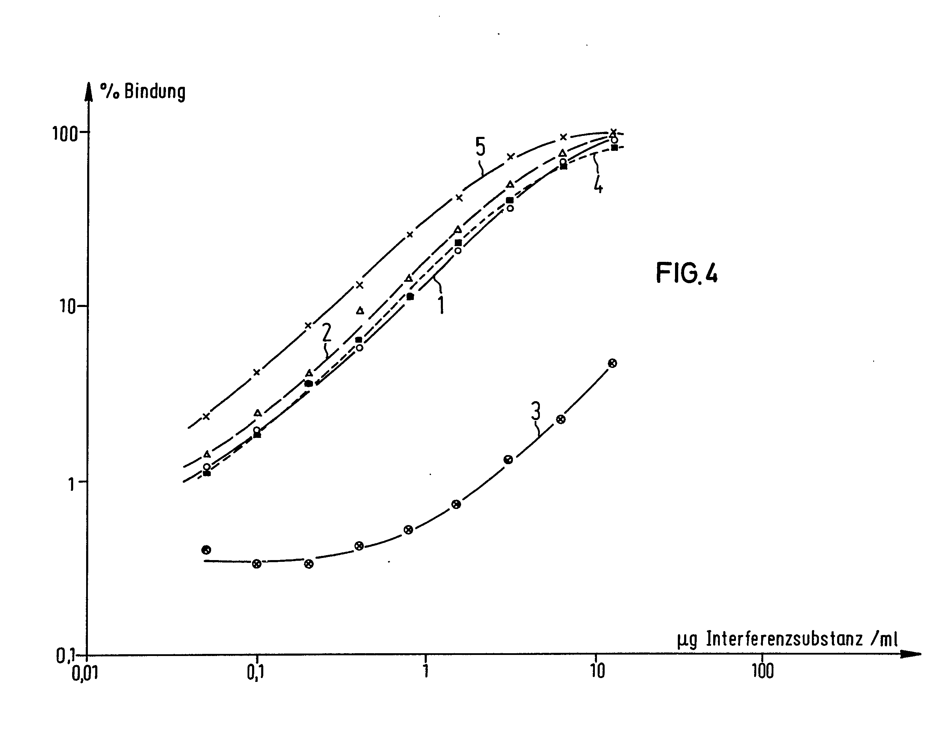

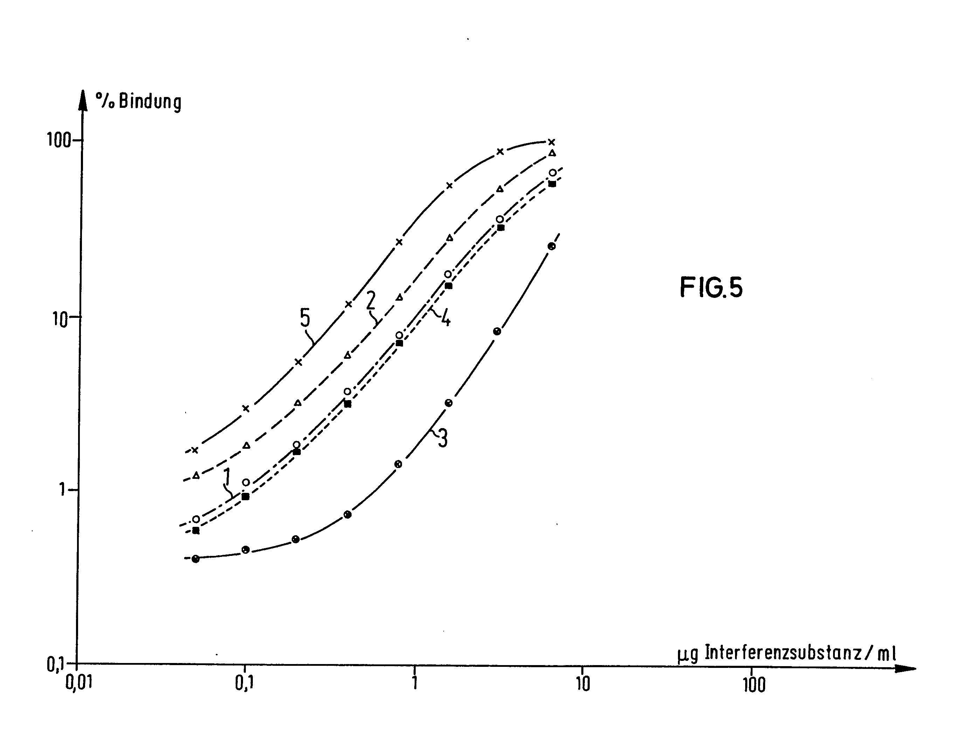

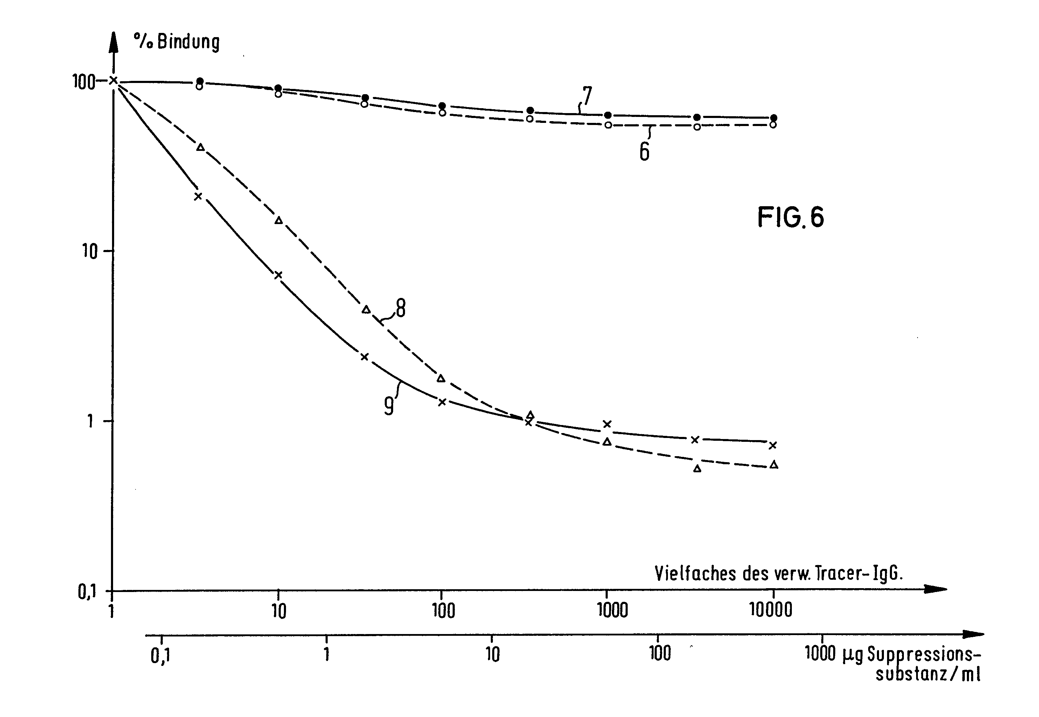

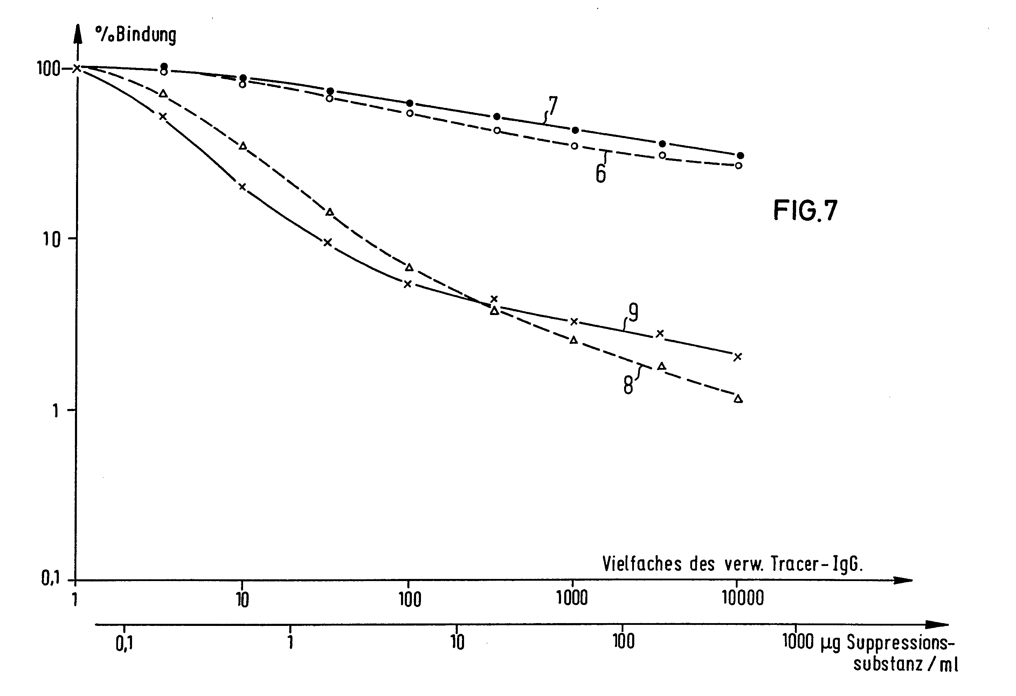

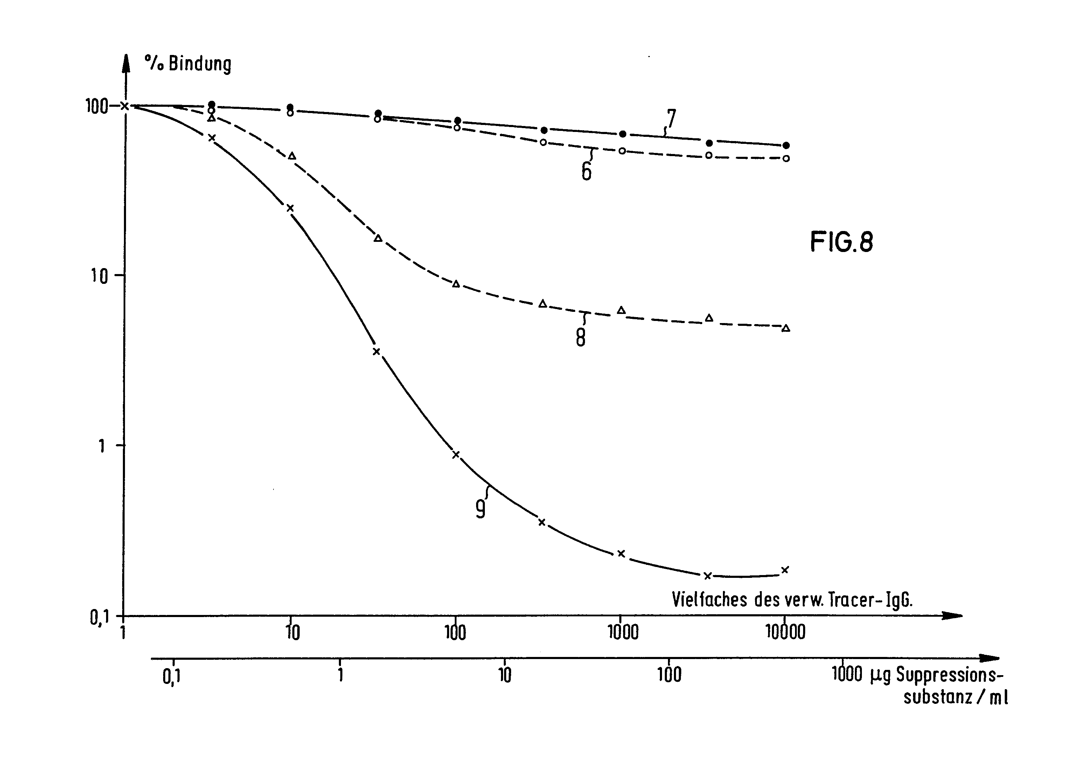

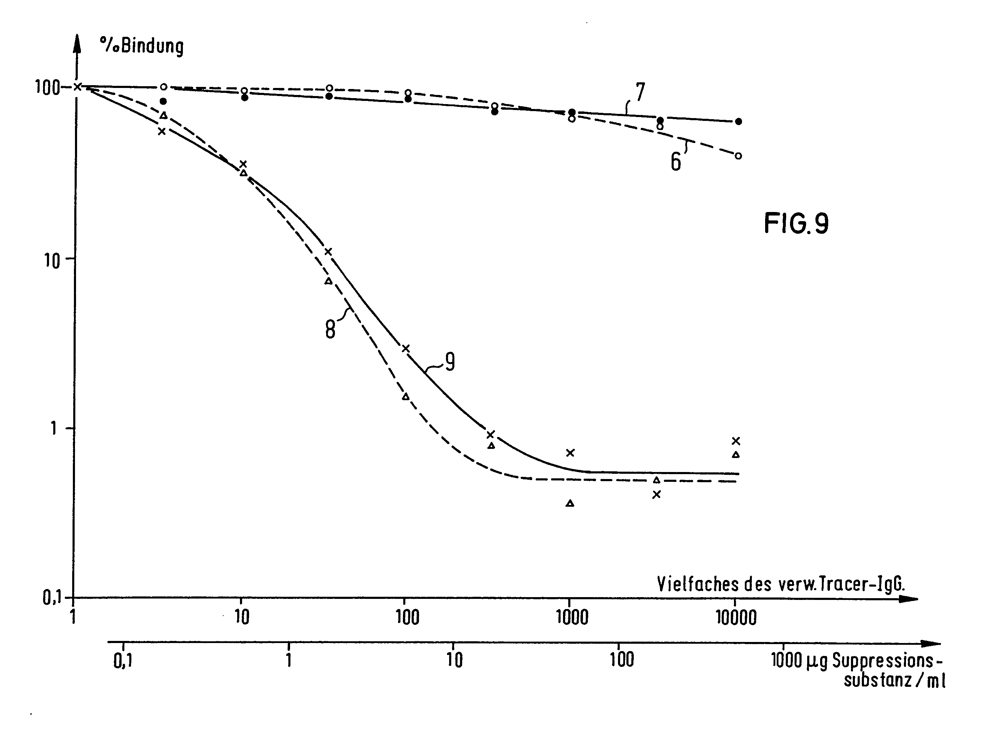

Die Bindungsaffinität von interferierenden Substanzen (Fig. 4: Anti-Maus-IgG aus der Ziege; Fig.5: Anti-Maus-IgG aus dem Kaninchen) zu den eingesetzten Reagenzantikörpern -ausgedrückt in % Bindung (100 % Bindung $\triangleq$ 100 % Reagenzantikörper ist an die interferierende Substanz gebunden) - wurde bei konstanter Menge an Suppressions-Medium in Abhängigkeit von der zugegebenen Menge an interferierender Substanz bestimmt. Es wurde das Testbesteck RIA-gnost [R] hTSH (Behringwerke, Marburg) verwendet. Als vergleichende Suppressions-Medien wurden

| | Kurve Nr. | (in Fig. IV und V) Vielfaches* | |
|---|---|---|---|
| Ratten-Serum | 1 | 4300 | 4300 |
| Ratten-IgG | 2 | 100 | 100 |
| erf.gem. Substanz (aus Bsp. 2) | 3 | 100 | 100 |

* bezogen auf das verwendete spezifische Tracer IgG

verwendet. Zum Vergleich sind in den Figuren IV und V noch zwei weitere Kurven eingezeichnet, die zum einen (Kurve Nr. 4) das zur Zeit auf dem Markt befindliche Testbesteck RIA-gnost hTSH beschreiben (enthält das 4400-fache eines unspezifischen Suppressions-IgG, bezogen auf das verwendete Tracer-IgG) und zum anderen ein RIA-gnost Testbesteck beschreiben, das frei von jeglichem Suppressionsmedium ist (Kurve Nr. 5, Vielfaches = 0; Standard).

Mit steigender Konzentration an Interferenz-Substanz (verwendet wurde: Anti-Maus-IgG aus der Ziege (Fig. IV) und Anti-Maus IgG aus dem Kaninchen (Fig. V)) steigt die Bindung (in %) an den Reagenzkörper. Es wird Analyt vorgetäuscht. Bei Verwendung der erfindungsgemäßen Substanzen aus Beispiel 2 ist der Anstieg der unspezifischen Bindungen am geringsten (Kurve Nr. 3 in Fig. IV und V).

Beispiel 6

Die prozentuale Bindung der Reagenzantikörper an eine interferierende Substanz, deren Konzentration (Titer) konstant war, wurde in Abhängigkeit unterschiedlicher Mengen an Suppressions-Medium bestimmt. Als interferierende Substanzen wurden verwendet:

|  | Titer |  |
|---|---|---|
| Anti-Maus-IgG aus der Ziege | 1,5 µg/ml | (Fig. VI) |
| Anti-Maus IgG aus dem Kaninchen | 1,5 µg/ml | (Fig. VII) |
| Patientenserum A |  | (Fig. VIII) |
| Patientenserum B |  | (Fig. IX) |

Als vergleichende Suppressions-Substanzen wurden verwendet:

|  | Kurve Nr. in Fig. VI bis IX |
|---|---|
| Ratten IgG | 6 |
| Ratten-Serum | 7 |
| Maus-IgG | 8 |
| erf.gem. Substanz (aus Bsp. 2) | 9 |

## Ansprüche

1. Immunometrisches Bestimmungsverfahren für eine mindestens zwei Antikörperbindungsstellen aufweisende antigene Substanz, bei dem man eine die antigene Substanz (a) enthaltende Flüssigkeitsprobe mit einem auf einer Festphase immobilisierten, unmarkierten, für (a) spezifischen Antikörper (b) und einem weiteren für (a) spezifischen, markierten Antikörper (c) sequentiell oder in einem Schritt inkubiert, wobei sich ein festphasengebundener, ternärer Komplex aus (a), (b) und (c) bildet, der ein nachweisbares Signal entsprechend der Menge von (a) aussendet, dadurch gekennzeichnet, daß bei dem sequentiellen Verfahren im ersten und/oder zweiten Inkubationsschritt, bei dem 1-Schritt-Verfahren zu dem Reaktionsgemisch, Protein zugesetzt wird, welches den verwendeten Antikörpern immunologisch verwandt ist.

2. Verfahren nach Anspruch 1, dadurch gekennzeichnet, daß es sich bei dem Protein um einen oder mehrere verschiedene Antikörper handelt.

3. Verfahren nach Anspruch 2, dadurch gekennzeichnet, daß es sich bei dem oder den Antikörpern um Antikörper der gleichen Spezies wie die Reagenzantikörper handelt.

4. Verfahren nach Anspruch 3, dadurch gekennzeichnet, daß es sich um einen monoklonalen oder mehrere monoklonale Antikörper handelt.

5. Verfahren nach Anspruch 4, dadurch gekennzeichnet, daß es sich bei dem oder den monoklonalen Antikörpern um monoklonale, anti-idiotypische Antikörper handelt.

6. Verfahren nach Anspruch 3, dadurch gekennzeichnet, daß der oder die monoklonalen Antikörper bis auf die Antigenbindungsstelle mit den Reagenzantikörpern übereinstimmen.

7. Verfahren nach Anspruch 6, dadurch gekennzeichnet, daß die Antigenbindungsstellen der Reagenzantikörper immunologisch blockiert werden.

8. Verfahren nach Anspruch 7, dadurch gekennzeichnet, daß zur Blockade ein monoklonaler, anti-idiotypischer Antikörper verwendet wird.

9. Verfahren nach Anspruch 6, dadurch gekennzeichnet, daß die Antigenbindungsstellen der Reagenzantikörper durch Mutation in Zellkulturen verlorengegangen sind und durch geeignetes Screening diese Antikörper herausgesucht werden.

10. Verfahren nach Anspruch 6, dadurch gekennzeichnet, daß die Antigenbindungsstellen der Reagenzantikörper chemisch verändert werden oder selektiv abgespalten werden.

11. Verfahren gemäß Anspruch 2, dadurch gekennzeichnet, daß der oder die zugesetzten Antikörper mindestens in einem zehnfachen Überschuß zu den Reagenzantikörpern eingesetzt werden.

12. Verfahren nach Anspruch 1, dadurch gekennzeichnet, daß der auf der Festphase immobilisierte, unmarkierte Antikörper (b) ein monoklonaler oder polyklonaler Antikörper ist.

13. Verfahren nach Anspruch 12, dadurch gekennzeichnet, daß der unmarkierte Antikörper (b) an die Festphase adsorptiv oder kovalent gebunden ist.

14. Verfahren nach Anspruch 1, dadurch gekennzeichnet, daß die Festphase aus einem Kunststoffröhrchen, Mikrotiter-Platten, Kunststoffkugeln oder -propellern oder aus mikroskopisch kleinen Kunststoffkugeln besteht, die in einer Flüssigkeit suspendiert sind.

15. Verfahren nach Anspruch 1, dadurch gekennzeichnet, daß der markierte Antikörper (c) in monoklonaler oder polyklonaler Antikörper ist und ein radioaktives Isotop, ein Enzym, ein fluoreszierende oder eine chemilumineszierende Gruppe als Indikator trägt.

16. Verfahren nach Anspruch 1, dadurch gekennzeichnet, daß als antigene Substanz das humane thyreoideastimulierende Hormon (hTSH) oder die onkofetalen Proteine Alfa-Fetoprotein, h-Choriongonadotropin oder das carcinoembryonale Antigen bestimmt werden.

FIG.1

FIG. 2

Signal

NSB

SB

Analyt-
konzentr.

normal ←→ pathologisch

FIG.3

FIG.4

FIG.5

EP 0 296 544 A2

FIG.6

FIG.7

FIG.8

% Bindung

100

10

1

0,1

Vielfaches des verw. Tracer-IgG.

1    10    100    1000    10000

0,1    1    10    100    1000 µg Suppressions-
substanz / ml

7

6

8

9

EP 0 296 544 A2

FIG.9

EP 0 296 544 A2